## Europäisches Patentamt
## European Patent Office
## Office européen des brevets

(11) Publication number: **0 196 902**
**A2**

(12)
# EUROPEAN PATENT APPLICATION

(21) Application number: **86302364.4**

(22) Date of filing: **27.03.86**

(51) Int. Cl.⁴: **C 07 C 2/28**
**C 07 C 41/01, C 07 C 29/04**
**C 10 L 1/02**

(30) Priority: **27.03.85 GB 8507971**

(43) Date of publication of application:
**08.10.86 Bulletin 86/41**

(84) Designated Contracting States:
**AT BE CH DE FR GB IT LI LU NL SE**

(71) Applicant: **The British Petroleum Company p.l.c.**
**Britannic House Moor Lane**
**London EC2Y 9BU(GB)**

(72) Inventor: **Atkins, Martin Philip**
**The British Petroleum Co. p.l.c. Chertsey Road**
**Sunbury-on Thames Middlesex, TW16 7LN(GB)**

(72) Inventor: **Westlake, David Jack**
**The British Petroleum Co. p.l.c. Britannic House**
**Moor Lane London, EC2Y 9BU(GB)**

(72) Inventor: **Gregory, Reginald**
**The British Petroleum Co. p.l.c. Chertsey Road**
**Sunbury-on-Thames Middlesex, TW16 7LN(GB)**

(74) Representative: **Crack, Richard David et al,**
**BP INTERNATIONAL LIMITED Patents Division Chertsey**
**Road**
**Sunbury-on-Thames Middlesex TW16 7LN(GB)**

(54) **Process for the hydration and oligomerisation of olefins.**

(57) A product mixture containing oligomers of alkenes, ethers and optionally alcohols is prepared by the hydration and oligomerisation of the alkenes by passing the alkenes and water in a molar ratio of alkenes to water of about 3:1 to 90:1 under hydration conditions over an acid hydration catalyst, particularly a cation exchange resin. The process can be controlled so as to produce less than 0.5% by wt water in the liquid reaction product, at least 40% by wt of oligomers and less than 5% by wt of material of $C_{12}$ and higher.

EP 0 196 902 A2

PROCESS FOR THE HYDRATION AND OLIGOMERISATION OF OLEFINS

This invention relates to a process for the preparation of a composition suitable for addition to a motor gasoline which process comprises the hydration and oligomerisation of $C_3$ to $C_6$ alkenes.

It has been previously proposed to incorporate oxygen-containing compounds into motor gasoline for a variety of different purposes. For example, to increase the amount of useful fuel, methanol has been described. To improve octane number, methyl tertiary butyl ether has been proposed. Oxygen-containing compounds have also been described for other purposes, for example, French Patent No 2149113 discloses the use of aliphatic ethers, optionally in combination with an alcohol, as a gasoline additive for reducing carbon monoxide emissions and isopropanol has been described as a carburettor deicing additive.

French Patent 2 149 113 discloses the use of aliphatic ethers, including diisopropyl ether, optionally in combination with an alcohol, as a gasoline additive for reducing carbon monoxide emissions.

British Patent No 950 147 discloses a fuel composition which comprises (a) a major proportion of a hydrocarbon base boiling in the gasoline range and having a Research Octane Number of at least 90, (b) an octane improving amount of an organic metal containing anti-knock agent e.g. tetra-alkyl lead, (c) a co-anti-knock agent which is a monoester and (d) a co-anti-knock extender which is an alcohol or an ether. A suitable co-anti-knock extender is diisopropyl ether containing a minor proportion of isopropanol. The

1

organic metal containing anti-knock agent is an essential component of the compositions since it is stated in the patent that in the absence of the organic metal containing anti-knock agent substantially no octane number benefit is obtained by the addition of the co-anti-knock agents to the gasoline.

It is important that oxygen-containing compounds or mixtures added to gasoline should have high Blending Octane Numbers (BRON) when blended to typical gasoline basestocks.

A process has now been invented to which alkenes are converted to a product mixture of oligomers and ethers which has high BRON when blended in typical gasoline basestocks.

According to the present invention a process for the hydration and oligomerisation of one or more $C_3$ to $C_6$ olefins to produce a reaction product containing oligomers of the alkenes, ethers and optionally alcohols comprises passing a feed containing $C_3$ to $C_6$ alkenes and water in a molar ratio of water to alkene of from 1:3 to 1:90 over an acid hydration catalyst under hydration conditions.

The alkene for the feed can conveniently be provided by refinery process streams a substantial proportion of which comprise $C_3$ to $C_5$, more preferably $C_3$ and/or $C_4$, alkenes such as, for example, $C_3/C_4$ streams from catalytic cracking.

Any conventional acid hydration catalyst may be used. Suitable catalysts include (A) cation-exchangeable layered clay in which there is a metal cation, (B) hydrogen ion-exchanged layered clays, (C) stabilised pillared interlayered clay and (D) cation exchanged organic resins. Catalysts of the type (A), (B) and (C) are known and are described, for example, in published European Patent Applications 0031687, 0031252, 0083970 and 0116469. Catalysts of type (D) are also known and some are commercially available.

Preferably the acid hydration catalyst is a cation exchange organic resin.

Preferably the molar ratio of water to alkene in the feed is from 1:4 to 1:50 and more preferably in the case of a butene from 1:20 to 1:50 and in the case of a propene 1:5 to 1:20.

It has been found that the process has unexpected advantages in that it can be controlled so as (i) to produce a liquid reaction product which contains at least 40% by weight of oligomers but less than 5% by wt of products of carbon number 12 and higher and also (ii) to produce a liquid reaction product containing less than 0.5% by weight of water, preferably less than 0.2%. This latter advantage is particularly significant in that the product can be added direct to a gasoline basestock without any intermediate drying step.

The molar proportions of reactants and reaction conditions can be selected so as (i) to produce a liquid reaction product containing from 70 to 99% by weight of oligomers, from 1 to 30% by weight of ethers and up to 10% by weight of alcohols or (ii) to produce a liquid reaction product containing from 40 to 70% by weight of oligomers, from 20 to 50% by weight of ethers and from 10 to 40% by weight of alcohols.

The ethers are typical dialkyl ethers and at least one of the alkyl group is usually branched.

The liquid product can be added to any gasoline for example a straight run gasoline, a cat cracked spirit or mixtures thereof. Preferably the gasoline has, before the addition of the additive composition a Research Octane Number (RON) of from 80 to 105, more preferably 90 to 98, and a Motor Octane Number (MON) of from 75 to 95, more preferably from 80 to 88. The gasoline may contain the following proportions of olefins, aromatics and saturates:

| Aromatics | 20 to 60% volume |
| Saturates | 20 to 70% volume |
| Olefins | 0 to 30% volume |

The octane improving additive possesses high Blending Research Octane Number and Blending Motor Octane Number which generally increase with increasing amounts of oxygenated components. Typical values are in the range 100 to 116 for Research Octane Number and 95 to 106 for Motor Octane Number in one gasoline basestock. The Blending Octane Numbers obtained will vary with the composition of the gasoline used as basestock.

Conventional organic metal containing anti-knock agents such as tetra-ethyl lead are not required in gasolines incorporating products according to the present invention. However, other conventional gasoline additives such as, for example, scavengers and anti-oxidants may be included in the fuel compositions. Other oxygenated compounds may also be included in the fuel compositions according to the present invention.

The present invention is illustrated by the following examples.

Example 1  not according to the invention.

A mixture of propene and n-heptane was hydrated. The amount of propene in the mixture was 46% by weight and the molar ratio of propene to water was 1.5:1. The feedstock was passed in a continuous upward flow through a hydrogen exchanged Wyoming Bentonite catalyst. The process was carried out at a temperature of 158°C, and a liquid hourly space velocity (LHSV) of 2.

The catalyst was prepared by adding sodium bentonite (a Wyoming Bentonite supplied as a fine powder for use in drilling muds) to a solution of concentrated sulphuric acid (400ml) in water (1100ml) and allowing the mixture to stand at room temperature (approximately 20°C) for 48 hours with occasional stirring. The clay was separated from the solution and washed with water by repeated centrifuging and re-suspending in water until the pH of the supernatant solution was the same as the distilled water used in the washing. The clay was dried at 80°C in air and ground into granules.

A liquid sample produced by the process over a 16 hour period comprised 97% by weight of an organic phase and 3% by weight of an aqueous phase. The organic phase comprised 66.3% by weight heptane, 2.4% by weight isopropanol, 9.0% by weight diisopropyl ether and 22.3% by weight propene oligomers. The aqueous phase comprised approximately 18.4% by weight isopropanol. The conversion of propene was calculated to be 56% and the conversion of water was calculated to be 72%.

Example 2

An octane improving additive composition was prepared by hydrating a mixture of propene and propane. The molar ratio of propene to propane in the feedstock was 1:1 and the molar ratio of propene to water was 8:1. The feedstock was passed in a continuous downward flow over a catalyst bed of a commercially available catalyst sold by Dow under the trade name DOWEX 50 W-X8(H) which is a hydrogen ion-exchanged cation exchanged organic resin catalyst in which the active group is a sulphonic acid group. The hydration was carried out at a temperature of 147°C and a pressure of 60 bar. The liquid hourly space velocity (LHSV) was 1.5. Propene conversion was approximately 5%. The single phase liquid product obtained comprised 76% by weight ispropanol and 17% by weight diisopropyl ether. The product was combined with the product of other runs carried out under similar reaction conditions. This bulk mixture was partially dried using anhydrous magnesium sulphate. The octane improving additive composition produced comprised 70% by weight isopropanol, 22% by weight diisopropyl ether, 3% by weight propene oligomers and 5% water. The octane improving additive was blended with an unleaded gasoline having a Research Octane Number of 90 and a Motor Octane Number of 82. The concentration of the additive in the gasoline was 10% by volume. Using this pure composition, the Blending Research Octane Number of the octane improving additive composition was determined to be 110 and the Blending Motor Octane Number was 97.

Example 3

An octane improving additive composition suitable for use in fuel compositions was prepared by reacting water and a mixture comprising 43.8% by weight butene, 20.9% by weight n-butene, 8.2% by weight isobutene and 26.9% by weight propene over the same ion-exchanged resin catalyst as used in Example 2. The molar ratio of alkene to water was 4.5:1. The hydration reaction was carried out at a temperature of 150°C and a pressure of 80 bar. The liquid hourly space velocity was 2. The conversion of alkenes was 15% and the conversion of water was 36%. The liquid product comprised 51% by weight of alcohols, 8.4% ethers, 13% by weight oligomers, 5% dissolved gases and 22% water. This product was combined with the product obtained in other runs under similar conditions. After partial drying over anhydrous magnesium sulphate, the product

comprised 58.5% by weight alcohols, 18% by weight ethers, 18.2% by weight oligomers and 4% by weight water. The octane improving additive composition was blended with an unleaded gasoline having a Research Octane Number of 90 and a Motor Octane Number of 82. The concentration of the additive in gasolinewas 10% by volume. Using this fuel composition, the Blending Research Octane Number and the Blending Motor Octane Number of the octane improving additive composition were found to be 112 and 100 respectively.

Example 4

Example 3 was repeated except that the molar ratio of alkene to water was 4:1 and the catalyst used was a commercially available hydrogen ion-exchanged catalyst sold under the trade name NAFION 811. The conversion of alkene was 18% and the conversion of water was 92%. The liquid product comprised 27.3% by weight alcohols, 26.1% by weight ethers, 35.3% by weight oligomers, 8.9% dissolved gases and 2.3% by weight water. Using a 10% by volume blend of the composition in an unleaded gasoline having a Research Octane Number of 90 and a Motor Octane Number of 82, the Blending Research Octane Number and Blending Motor Octane Number of the octane improving additive composition were found to be 110 and 102 respectively.

Example 5  not according to the invention

Propene (40g) was charged into a stainless steel autoclave (volume 200ml) containing 10 grams of the same commercial ion-exchanged organic resin catalyst as used in Example 2. The catalyst contained 50% by weight of water to give a molar ratio of alkene to water of about 1:1. A further 10 grams of water were charged into the autoclave. The autoclave was sealed and stirred, by means of a sealed magnetic stirrer, at a reaction temperature of 160°C for a reaction period of 2.5 hours. After cooling, the gaseous products were vented off, and the liquid products removed. The yield and composition of the product are given in Table 5.

Example 6  not according to the invention

Example 5 was repeated except that only 2.5 grams of additional water were added to the autoclave instead of 10 grams to give a molar ratio of alkene to water of 2:1. The yield and composition of the liquid product are given in Table 5.

Example 7

Example 5 was repeated except that no additional water was added to the autoclave the molar ratio of alkene to water being 3.6:1. The yield and the composition of the of the liquid product are given in Table 5.

Table 5

| Example No | Yield of Liquid Product (g) | Composition of Liquid Product (%wt) | | | |
|---|---|---|---|---|---|
| | | Isopropanol | Diisopropyl Ether | Propene Oligomers | Water |
| 5 | 21.8 | 68 | 11 | - | 21 |
| 6 | 17.3 | 29 | 50 | 5 | 16 |
| 7 | 19.6 | 16 | 34 | 50 | - |

The results given in Table 5 show how the amount of water affects the compositions of the octane improving additive.

Example 8

The alkene containing feedstock for this example was a $C_3/C_4$ hydrocarbon stream produced by a catalytic cracker analysed by gas chromatography as having the composition shown in Table 6.

The feedstock together with water in the alkene to water molar ratio of 8:1, was passed in a continuous downward flow over a catalyst bed consisting of pieces of Nafion 811 tubing (a perfluorinated resin with active sulphonic acid groups commercially manufactured by DuPont de Nemours). The reaction temperature was 140°C rising to 160°C at the end of the catalyst bed. The reaction pressure was 80 bar gauge and the LHSV was 2. Under these conditions 51% of the alkenes were converted into a liquid product, produced by oligomerisation and hydration reactions, containing 10%

w/w dissolved $C_3/C_4$ feedstock, 71% low molecular weight oligomers of propene and butenes (53% $C_6$ to $C_9$ alkenes, 18% $C_{10}$ to $C_{12}$ alkenes, with only a trace of hydrocarbons with a carbon number greater than $C_{12}$ detected) and 18.4% oxygenates (0.4% acetone, 3.6% isopropanol, 0.1% tertiary-butanol, 2.0% secondary butanol, 7.3% diisopropyl ether, 0.3% isopropyl propyl ether, 4.0% isopropyl secondary butyl ether and 0.7% di-secondary-butyl ether) and 0.14% water.

The octane improving liquid was blended as a 10% volume additive in an unleaded gasoline having a Research Octane Number of 90 and a Motor Octane Number of 82. Using this fuel composition, the Blending Research Octane Number and the Blending Motor Octane Number of the octane improving composition were determined as 111.2 and 99.6 respectively.

Example 9

Example 8 was repeated but using a proportion of feedstock so that the alkenes:water molar ratio was 6:1 instead of 8:1. Under these conditions, a 25% conversion of alkenes was obtained. The liquid products contained 10% w/w dissolved $C_3/C_4$ feedstock, 56% low molecular weight oligomers of propene and butenes (44% $C_6$ to $C_9$ alkenes, 12% $C_{10}$ to $C_{12}$ alkenes, with no hydrocarbons of carbon number greater than $C_{12}$ detected), 32.4% oxygenates (0.4% acetone, 0.4% tertiary butanol, 3.7% secondary butanol, 11.2% diisopropyl ether, 0.9% isopropyl propyl ether, 6.5% isopropyl secondary butyl ether, and 0.7% di-secondary-butyl ether) and 0.76% water.

The octane improving liquid was blended as a 10% volume additive in an unleaded gasoline having a Research Octane Number of 90 and a Motor Octane Number of 82. Using this fuel composition, the Blending Research Octane Number and the Blending Motor Octane Number of the octane improving composition was determined as 115.2 and 102.6.

Example 10

Example 8 was repeated but using XE-386 as catalyst instead of Nafion 811. XE-386 is an ion-exchange resin manufactured by Rohm and Haas which is a polystyrene divinyl benzene copolymer with active sulphonic acid groups. The catalyst was dried at 80°C for 16 hours before use.

A feedstock comprising the composition shown in Table 6 and water with an alkenes:water molar ratio of 7:1 was fed in a similar manner to Example 9. The reaction temperature was 139°C rising to 142°C at the end of the catalyst bed. The reaction pressure was 80 bar gauge and the LHSV 2. Under these conditions, 54% of the alkenes were converted into a liquid product containing 15% dissolved $C_3/C_4$ feedstock, 57% low molecular weight oligomers of propene and butenes (50% $C_5$ to $C_9$ alkenes, 7% $C_{10}$ to $C_{12}$ alkenes, and no hydrocarbons with carbon number greater than $C_{12}$ were detected), 28% oxygenates (6.0% isopropanol, trace of tertiary butanol, 3.4% secondary butanol, 10.0% diisopropyl ether, 1.9% isopropyl propyl ether, 5.9% isopropyl secondary butyl ether and 0.8% di-secondary-butyl ether) and 0.15% water.

The octane improving liquid was blended as a 10% volume additive in an unleaded gasoline having a Research Octane Number of 90 and a Motor Octane Number of 82. Using this fuel composition, the Blending Research Octane Number and the Blending Motor Octane Number of the octane improving composition were determined as 116.3 and 99.8 respectively.

Example 11

Example 10 was repeated but using a molar ratio of alkenes to water of 12:1 and a reaction temperature of 119 to 149°C instead of 7:1 and 139 to 142°C respectively. Under these conditions, 63% of the alkenes are converted into a liquid product containing 17% w/w dissolved $C_3/C_4$ feedstock, 73% low molecular weight oligomers of propene and butenes (53% $C_5$ to $C_9$ alkenes, 20% $C_{10}$ to $C_{12}$ alkenes, with only a trace of hydrocarbons with carbon number greater $C_{12}$ being detected), 10% oxygenates (0.5% isopropanol, a trace of tertiary butanol, 0.6% secondary butanol, 4.9% diisopropyl ether, 0.1% isopropyl propyl ether, 3.2% isopropyl secondary butyl ether, and 0.7% di-secondary-butyl ether), and 0.03% water.

The octane improving liquid was blended as a 10% volume additive in an unleaded gasoline having a Research Octane Number of 90 and a Motor Octane Number of 82. Using this fuel composition, the Blending Research Octane Number and the Blending Motor Octane Number were determined as 104.0 and 99.3 respectively.

Example 12

Example 10 was repeated but using a molar ratio of alkenes to water of 15:1 and a reaction temperature of 125 to 142°C instead of 7:1 and 139 to 142°C respectively. Under these conditions, 70% of the alkenes were converted to liquid products containing 18% dissolved $C_3/C_4$ feedstock, 74% low molecular weight oligomers of propene and butenes (56% $C_5$ to $C_9$ alkenes, 18% $C_{10}$ to $C_{12}$ alkenes, and less than 1% of greater than $C_{12}$ hydrocarbons were detected), 7% oxygenates (0.1% acetone, 0.6% isopropanol, a trace of tertiary butanol, 0.5% secondary butanol, 3.7% diisopropyl ether, 1.8% isopropyl secondary-butyl ether and 0.3% di-secondary-butyl ether) and 0.03% water.

Example 13

Example 11 was repeated but using a reaction pressure of 30 bars gauge instead of 80 bars gauge and a reaction temperature of 126 to 153°C instead of 119 to 149°C. Under these conditions, 55% of the alkenes were converted to liquid products containing 14% w/w dissolved $C_3/C_4$ feedstock, 75% low molecular weight oligomers of propene and butenes, 11% oxygenates (ether:alcohol weight of propene and butenes (57% $C_5$ to $C_9$ alkenes, 18% $C_{10}$ to $C_{12}$ alkenes with only a trace of hydrocarbons of carbon number greater than $C_{12}$ being detected), 11% oxygenates (0.6% acetone, 1.2% isopropanol, a trace of tertiary butanol, 0.8% secondary butanol, 5.4% diisopropyl ether, 2.6% isopropyl secondary-butyl ether and 0.4% di-secondary-butyl ether), and 0.04% water.

Example 14

Example 13 was repeated but using a reaction temperature of 113 to 130°C instead of 119 to 149°C. Under these conditions 64% of the alkenes were converted to liquid products containing 13% w/w dissolved $C_3/C_4$ feedstock, 77% low molecular weight oligomers of propene and butenes (64% $C_5$ to $C_9$ alkenes, 13% $C_{10}$ to $C_{12}$ alkenes, with no hydrocarbons with carbon number greater than $C_{12}$ being detected), 10% oxygenates (0.4% acetone, 0.1% isopropanol, 0.2%

0196902

secondary butanol, 6.3% diisopropyl ether, 2.7% isopropyl secondary-butyl ether, and 0.3% di-secondary-butyl ether) and 0.03% water.

Example 15

Example 11 was repeated but using a reaction temperature of 103 to 123°C instead of 119 to 149°C. Under these conditions 58% of the alkenes were converted to liquid products containing 11% dissolved unchanged $C_3/C_4$ feedstock, 76% low molecular oligomers of propene and butenes (65% $C_5$ to $C_6$ alkenes, 11% $C_{10}$ to $C_{12}$ alkenes and no higher carbon numbered hydrocarbons were detected) 13% oxygenates (0.4% acetone, 1.3% isopropanol, 0.3% secondary butanol, 0.1% isopropyl propyl ether, 3.5% isopropyl secondary butyl ether, and 0.5% di-secondary-butyl ether) and 0.03% water.

Example 16

Amberlite 252, a commercially available Rohm and Haas ion-exchange resin was added to a large excess of 2N sulphuric acid and left for 4 hours. The ion-exchange resin was filtered off and washed with water until acid free to give Amberlite 252 in the sulphonic acid form. The catalyst was dried at 80°C for 4 hours before use.

Example 8 was repeated but the catalyst was acid-exchanged Amberlite 252 instead of Nafion 811. The alkenes:water feedstock molar ratio was 5:1 instead of 8:1 and the reaction temperature was 134 to 143°C instead of 140 to 160°C. Under these conditions, 17% of the alkenes were converted to liquid products containing 14% w/w dissolved unchanged $C_3/C_4$ feedstock, 43% low molecular weight oligomers of propene and butenes (40% $C_5$ to $C_9$ alkenes, and 3% $C_{10}$ to $C_{12}$ alkenes with no hydrocarbon of above carbon number 12 being detected), 43% oxygenates (0.3% acetone, 9.8% isopropanol, 2.0% tertiary butanol, 3.7% secondary butanol, 16.5% diisopropyl ether, 1.2% isopropyl propyl ether, 8.5% isopropyl secondary butyl ether, and 1.0% di-secondary-butyl ether) and 0.2% water.

The octane improving liquid was blended as a 10% volume additive in an unleaded gasoline having a Research Octane Number of 90 and a Motor Octane Number of 82. Using this fuel composition,

**0196902**

the Blending Research Octane Number and the Blending Motor Octane Number were determined as 114.4 and 99.2 respectively.

Example 17

Example 8 was repeated but the catalyst was Amberlyst 17, a Rohm and Haas experimental ion-exchange resin with enhanced thermal stability instead of Nafion 811. The catalyst was dried at 80°C for 4 hours before use. The reaction temperature was 135 to 147°C instead of 140 to 160°C. Under these conditions 35% of the alkenes were converted to liquid products containing 14% w/w dissolved unchanged $C_3/C_4$ feedstock, 71% low molecular weight oligomers of propene and butenes (56% $C_5$ to $C_9$ alkenes, and 15% $C_{10}$ to $C_{12}$ alkenes with only a trace of hydrocarbons above carbon number 12 being detected), 15% oxygenates (0.3% acetone, 1.6% isopropanol, 1.0% secondary butanol, 7.7% diisopropyl ether, 0.3% isopropyl propyl ether, 3.6% isopropyl-secondary butyl ether, and 0.5% di-secondary-butyl ether). The water content was not analysed.

Example 18

Example 8 was repeated but the catalyst used was M31 instead of Nafion 811. M31 is a ion-exchange resin with octine sulphonic acid groups, manufactured by the Dow Chemical Company and has high thermal stability. The catalyst was dried at 80°C before use. The alkene:water feedstock molar ratio was 5:1 instead of 8:1, and the reaction temperature was 142 to 145°C instead of 140 to 160°C. Under these conditions, 24% of the alkenes were converted into a liquid product containing 12% w/w dissolved unchanged $C_3/C_4$ feedstock, 49% w/w low molecular weight oligomers of propene and butenes (40% $C_5$ to $C_9$ alkenes, and 9% $C_{10}$ to $C_{12}$ alkenes, no hydrocarbons were detected with carbon number higher than 12), 39% oxygenates (9.3% isopropanol, 1.0% tertiary butanol, 4.0% secondary butanol, 12.8% diisopropyl ether, 1.9% isopropyl propyl ether, 8.7% isopropyl secondary butyl ether, and 1.3% di-secondary-butyl ether). The water contect was not analysed.

Example 19

The alkene containing feedstock for this example is a $C_3$ hydrocarbon stream produced by a catalytic cracker analysed by gas chromatography as having the composition shown in Table 7.

A feedstock comprising the composition shown in Table 7 and water in a propene to water molar ratio of 9:1 was passed in a continuous downward flow over a catalyst bed consisting of pieces of Nafion 811 tubing. The reaction temperature was 132°C rising to 145°C at the end of the catalyst bed. The reaction pressure was 80 bar, and the LHSV 2. Under these conditions 36% of the propene was converted to liquid products containing 26% w/w dissolved unchanged feedstock, 70% low molecular weight oligomers of propene ($63\%$ $C_5$ to $C_9$ alkenes, $7\%$ $C_{10}$ to $C_{12}$ alkenes and no hydrocarbons with carbon number higher than 12 was detected) and 27.4% oxygenates (0.2% acetone, 2.0% isopropanol, 24.5% diisopropyl ether, and 0.7% isopropyl propyl ether) and no water was detected.

The octane improving liquid was blended as a 10% volume additive in an unleaded gasoline having a Research Octane Number of 90 and a Motor Octane Number of 82. Using this fuel composition, the Blending Research Octane Number and the Blending Motor Octane Number were determined as 101.1 and 94.3 respectively.

Example 20

Example 19 was repeated but using M31 as catalyst instead of Nafion 811. The propene to water molar ratio of the feedstock was 7:1 and the reaction temperature was 133°C rising to 147°C at the end of the catalyst bed instead of 9:1 and 132 to 145°c respectively. Under these conditions, 34% of the propene was converted to liquid products containing 2% w/w dissolved unchanged feedstock, 44% low molecular weight oligomers of propene ($40.3\%$ $C_5$ to $C_9$ alkenes and $3.7\%$ $C_{10}$ to $C_{12}$ alkenes and no hydrocarbons with a carbon number higher than 12 were detected), 54% oxygenates (5.6% isopropanol, 48.2% diisopropyl ether and 0.2% isopropyl propyl ether) and 0.5% water.

The octane improving liquid was blended as a 10% volume additive in an unleaded gasoline having a Research Octane Number of 90 and a Motor Octane Number of 82. Using this fuel composition, the Blending Research Octane Number and the Blending Motor Octane Number was determined as 106.4 and 99.2 respectively.

Example 21

The alkene containing feedstock for this example was a $C_4$ hydrocarbon stream produced by a steam cracker after butadiene and the bulk of the isobutene has been consumed by other processes. It has been analysed by gas chromatography as having the composition shown in Table 8.

A feedstock comprising the composition shown in Table 8 and water in an alkene to water mole ratio of 42:1 was passed in a downward flow over a catayst bed consisting of XE-386 ion-exchange resin. The reaction temperature was 126°C rising to 146°C at the end of the catalyst bed. The reaction pressure was 80 bar and the LHSV was 2. Under these conditions, 54% of the alkenes were converted to liquid products containing 33% w/w dissolved unchanged $C_4$ feedstock, 63% low molecular weight oligomers of butenes (with a $C_8:C_{12}$ alkene weight ratio of 20:1 and no higher oligomers than $C_{12}$ were detected), and 2.1% oxygenates (0.6% secondary butanol and 1.5% di-secondary-butyl ether) and no water was detected.

TABLE 6

COMPOSITION OF THE $C_3/C_4$ CAT CRACKER STREAM

| Component | Composition (% w/w) |
|---|---|
| Ethane/Ethylene | 0.2 |
| Propane | 7.4 |
| Propene | 27.3 |
| Isobutane | 20.3 |
| n-Butanes | 5.1 |
| trans-But-2-ene | 10.7 |
| But-1-ene | 9.9 |
| Isobutene | 12.0 |
| cis-But-2-ene | 7.2 |
| Pentanes | trace |

TABLE 7

COMPOSITION OF THE $C_3$ CAT CRACKER STREAM

| Component | % Weight |
|---|---|
| Ethane | 0.7 |
| Propane | 20.2 |
| Propene | 76.8 |
| Isobutene | 1.8 |
| Butenes | 0.5 |

TABLE 8

COMPOSITION OF THE C$_4$ STEAM CRACKER STREAM

| Component | % Weight |
|-----------|----------|
| n-Butanes | 22.7 |
| Isobutane | 4.4 |
| But-1-ene | 55.1 |
| But-2-ene | 16.3 |
| Isobutene | 1.3 |
| Pentanes | 0.1 |

Claims:

1. A process for the hydration and oligomerisation of one or more $C_3$ to $C_6$ alkenes to produce a reaction product containing oligomers of the alkenes, ethers and optionally alcohols, which process comprises passing a feed containing one or more $C_3$ to $C_6$ alkenes and water in a molar ratio of water to alkene of from 1:3 to 1:90 over an acid hydration catalyst under hydration conditions.

2. A process as claimed in claim 1 wherein the acid hydration catalyst is a cation exchange organic resin.

3. A process as claimed in claim 1 or 2 wherein the process is controlled such that the water content of the liquid reaction product is less than 0.5% by weight.

4. A process as claimed in any one of the preceding claims wherein the conditions are controlled such that the liquid reaction product contains at least 40% by weight of oligomers.

5. A process as claimed in any one of the preceding claims wherein the molar ratio of water to alkene in the feed is from 1:8 to 1:20.

6. A process as claimed in any one of the preceding claims wherein the feed comprises $C_3$ and/or $C_4$ alkenes and the conditions are controlled such that the content of products of carbon number 12 and higher is less than 5% by weight.

7. A process as claimed in any one of the preceding claims wherein the temperature is within the range 120 to 180°C, the pressure from 20 to 100 bar absolute and the LHSV of feed from 0.5 to 10.

8. A process as claimed in any one of claims 2 to 7 wherein the cation exchange organic resin contains sulphonic acid groups.

9. A process as claimed in any one of the preceding claims wherein the molar proportions of reactants and the reaction conditions are selected so as to produce a reaction product containing from 70 to 99% by weight of oligomers from 1 to 30% by weight of esters and up to 10% by weight of alcohols.

10. A process as claimed in any one of claims 1 to 8 wherein the molar proportions of reactants and reaction conditions are selected so as to produce a reaction product containing from 40 to 70% by weight of oligomers, from 20 to 50% by weight of ethers and from 10 to 40% by weight of alcohols.

11. A composition suitable for addition to a motor gasoline containing oligomers, ethers and optionally alcohols and wherein the amount of oligomers is from 70 to 99% by weight, the amount of ethers from 1 to 30% by weight and the amount of $C_3$ to $C_6$ alcohols from 0 to 10% by weight, the weight being based on the weight of the total composition.

12. A composition suitable for addition to a motor gasoline containing oligomers, ethers and alcohols and wherein the amount of oligomers is from 40 to 70% by weight the amount of ethers is from 20 to 50% by weight and the amount of alcohols from 10 to 40% by weight the weight being based on the weight of the total composition.

13. A composition as claimed in claim 11 or 12 which composition contains less than 0.5% by weight of water.

14. A composition as claimed in any one of claims 11 to 13 wherein the composition contains less than 0.2% by weight of water.

15. A motor gasoline comprising (i) at least 60% by weight of a gasoline basestock, and (ii) a composition as claimed in any one of claims 11 to 14.

16. A motor gasoline containing from 80 to 95% by weight of a gasoline basestock and from 5 to 20% by weight of a composition as claimed in any one of claims 11 to 14.

17. A motor gasoline composition comprising

(1) from 60 to 95% by weight of gasoline range hydrocarbons

(ii) from 0 to 16% by weight of $C_3$ to $C_6$ alcohols

(iii) from 1 to 20% by weight of one or more branched ethers boiling below 200°C, and

(iv)  from 4 to 39% by weight of oligomers of $C_3$ to $C_6$ allkenes boiling in the gasoline range.

18.  A motor gasoline as claimed in claim 17 comprising

(i)  from 80 to 95% by weight of gasoline range hydrocarbons

(ii)  from 1 to 10% by weight of $C_3$ to $C_6$ alcohols

(iii) from 1 to 10% by weight of one or more branched ethers boiling below 200°C and

(iv)  from 4 to 20% by weight of oligomers of $C_3$ to $C_6$ alkenes boiling in the gasoline range.

19.  A motor gasoline composition as claimed in any one of claims 15 to 18 wherein the branched ether is selected from one or more of diisopropyl ether, methyl tertiary butyl ether, disecondary butyl ether, isopropyl secondary butyl ether and tertiary amyl-methylether.